# EUROPEAN PATENT SPECIFICATION

(11) **EP 3 220 934 B1**
(45) Date of publication and mention of the grant of the patent: **12.06.2019**
(21) Application number: 15820643.3
(22) Date of filing: 23.11.2015
(51) Int. Cl.: A61K 36/81, A61K 31/00, A61K 31/01, A61K 31/575

(54) **BIOLOGICALLY-ACTIVE TOMATO COMPOSITION HAVING REDUCED AMOUNT OF LYCOPENE**
BIOLOGISCH AKTIVE TOMATENZUSAMMENSETZUNG MIT REDUZIERTER LYCOPINMENGE
COMPOSITION DE TOMATE BIOLOGIQUEMENT ACTIVE COMPORTANT UNE QUANTITÉ RÉDUITE DE LYCOPÈNE

(30) Priority: 25.11.2014 US 201462083909 P
(43) Date of publication of application: 27.09.2017
(73) Proprietor: Lycored Ltd., 84102 Beer Sheva (IL)
(72) Inventor: ZELKHA, Morris, 52232 Ramat-Gan (IL); SEDLOV, Tanya, 84496 Beer-Sheva (IL); SHARONI, Yoav, 84965 Omer (IL); LEVY, Joseph, 84965 Omer (IL)
(74) Representative: Turner, Craig Robert
(86) International application number: PCT/IL2015/051129
(87) International publication number: WO 2016/084068

(56) References cited:
- WO-A2-2007/046083
- US-A1- 2012 258 182
- US-B1- 6 383 474
- Olivier Aust ET AL: "Supplementation with Tomato- Based Products Increase Lycopene, Phytofluene, and Phytoene Levels in Human Serum and Protects Against UV-light-induced Erythema", Int. J. Vitam. Nutr. Res., 1 January 2003 (2003-01-01), pages 1-7, XP55256471, DOI: 10.1024/0300-9831.74.1.??? Retrieved from the Internet: URL:http://www.xtend-life.com/docs/default -source/pdf/premium-fish-oil/study-lycopen e-reduces-skin-readness.pdf?sfvrsn=2 [retrieved on 2016-03-08]
- ANN G. LIU ET AL: "Feeding Tomato and Broccoli Powders Enriched with Bioactives Improves Bioactivity Markers in Rats", JOURNAL OF AGRICULTURAL AND FOOD CHEMISTRY, vol. 57, no. 16, 26 August 2009 (2009-08-26), pages 7304-7310, XP055255821, US ISSN: 0021-8561, DOI: 10.1021/jf901926b
- Anonymous: "CFR - Code of Federal Regulations Title 21", , 23 November 2010 (2010-11-23), XP055256202, Retrieved from the Internet: URL:https://web.archive.org/web/2010112306 3609/http://www.accessdata.fda.gov/scripts /cdrh/cfdocs/cfcfr/CFRSearch.cfm?fr=73.585 [retrieved on 2016-03-08]
- N. J. ENGELMANN ET AL: "Nutritional Aspects of Phytoene and Phytofluene, Carotenoid Precursors to Lycopene", ADVANCES IN NUTRITION: AN INTERNATIONAL REVIEW JOURNAL, vol. 2, no. 1, 1 January 2011 (2011-01-01) , pages 51-61, XP55255703, DOI: 10.3945/an.110.000075

## Description

### Field of the invention

The present invention relates to the field of food processing. Particularly, the invention relates to a biologically-active tomato composition having a reduced amount of lycopene.

### Background of the invention

Tomato products are widely used in both the food industry and, increasingly, in the preparation of nutraceutical and cosmeceutical compositions.

US 2012/258182 discloses pharmaceutical compositions for oral administration comprising a therapeutically effective amount of a synergistic combination of lycopene and at least one phytosterol wherein the ratio of said lycopene to said phytosterol in said pharmaceutical composition is a maximum of about 5:1 and wherein said composition produces a synergistic inhibition of cell growth.

US 5,837,311 describes an industrial process for the preparation of tomato-derived products including *inter alia* a tomato oleoresin containing high levels of the carotenoid lycopene. This oleoresin product also contains an impressively-large range of additional pharmacologically-active components including other carotenoids such as phytoene, phytofluene and beta-carotene, as well as tocopherols and phytosterols. This oleoresin product has been sold and used extensively and successfully for many years now, and the range of medical conditions that may be prevented and/or treated with it includes, but is not limited to, cancerous conditions (particularly prostate cancer), elevated blood pressure, atherosclerosis, skin conditions and cellular and DNA damage.

While this tomato oleoresin preparation has proven to be highly efficacious in the management of health and the prevention of disease, its striking red color may, in some circumstances, be disadvantageous, for example when its use on the skin as a cosmetic or cosmeceutical agent is contemplated.

It is an object of the present invention to provide a solution to this problem by providing a tomato-derived product having a similar (or improved) range of therapeutic activity to the above-mentioned tomato oleoresin that is known commercially as Lyc-O-Mato®, but which has a much less marked red color, by virtue of its low lycopene content.

Other aims and objectives of the invention will become apparent as the description proceeds.

### Summary of the invention

The present invention is primarily directed to a reduced-lycopene tomato-derived composition, comprising lycopene and one or both of phytoene and phytofluene, and phytosterols, wherein the concentration of said lycopene is in the range of 0.3% to 2% (weight/weight), wherein the weight ratio of said lycopene to one or both of phytoene and phytofluene is in the range of 1:1 to 1:2.5 and wherein the concentration of phytosterols is at least 2% (weight/weight).

In one preferred embodiment the above-disclosed composition further comprises vitamin E at a concentration of at least 2% (weight/weight).

In one preferred embodiment of the invention, the concentration of one or both of phytoene and phytofluene at a total concentration of between 0.25% and 3%.

In the context of the present disclosure, the terms "reduced-lycopene" and "low-lycopene" composition, and their like, are used interchangeably, and all of said terms are to be understood to refer to the composition having the carotenoid composition defined in the previous paragraph.

It is to be noted that the composition of the present invention possesses significant biological activity (including, but not limited to, anti-inflammatory activity, despite the fact that the lycopene content of said composition is reduced at least three-fold when compared with a commonly-used tomato oleoresin product of the prior art (Lyc-O-Mato®). This result is entirely unexpected, since many of the therapeutic effects of tomato oleoresins have been attributed to their lycopene content.

In one preferred embodiment, the composition of the present invention has a solvent concentration of less than 50ppm.

In some embodiments, the composition of the present invention may also comprise one or more additional pharmacologically-active components including, but not limited to, additional carotenoids, vitamin E and polyphenols, such as camosic acid. Preferred examples of additional carotenoids include phytoene, phytofluene, lutein, zeaxanthin, beta-carotene, astaxanthin. Other carotenoids, however, may also be present in the composition, either in addition to, or instead of, the examples listed above.

In one preferred embodiment of the invention, the reduced-lycopene composition further comprises camosic acid.

In another preferred embodiment, the reduced-lycopene composition further comprises soluble tomato solids.

In a further preferred embodiment the composition of the present invention further comprises beta-carotene and vitamin E. Preferably, the aforementioned additional components are present in the following concentration ranges: 0.2% -1.0% beta-carotene; 2.0% - 4% Vitamin E.

In one preferred embodiment, the above-disclosed composition is prepared from tomatoes.

In another aspect, the present invention provides a process for preparing the above-defined low-lycopene content, tomato-derived composition, wherein said process comprises the steps of:
1. Providing a tomato product comprising 5% - 20% Lycopene (for example, as described in US 5,837,311).
2. Adding a solvent or solvent mixture which dissolve the lipids and not the lycopene, including (but not limited to) ethanol, isopropyl alcohol (IPA) or acetone at a ratio higher than 1:1 (solvent : tomato product), preferably at a ratio of 3:1.
3. Heating the solvent/tomato extract mixture at a temperature of about 25° - 60°C.
4. Separating the crystalline material from the solvent by means of filtering and/or centrifugal separation.
5. Optionally incorporating the crystals, which contain approximately 70% lycopene, into colorant formulations.
6. Evaporating the solvent (which, in the case of ethanol, preferably leaves a liquor having less than 50 ppm ethanol), which may then be recycled for use in step 2.
7. Standardizing the resultant liquor to the required concentration, for example, by adding the feed composition.

In one preferred embodiment of the above-disclosed process, the solvent used in step 2 is ethanol.

In one preferred embodiment of the above-disclosed process, the solvent: tomato product ratio is 4:1.

While any suitable carotenoid-containing product derived from the tomato may be used as the starting material for this process, in one highly preferred embodiment, the tomato product used as the starting material is oleoresin. In another preferred embodiment, the tomato product used as the starting material comprises tomato peels.

The composition disclosed herein has been unexpectedly found, despite its greatly reduced lycopene concentration (in relation to prior art tomato oleoresins) to cause increased production of nitric oxide (NO) in vascular tissues. Furthermore, the present composition has been found to cause significant induction of the endothelial nitric oxide synthase enzyme in vascular endothelium.

The presently-disclosed composition has also been unexpectedly found to cause a significant increase in the activity of the antioxidant responsive element (ARE) system.

The presently-disclosed composition has further been unexpectedly found to cause significant inhibition of the pro-inflammatory NFkB transcription system.

The above-disclosed composition may be used as a medicament for treating many different types of medical conditions. In one preferred embodiment, said medicament is suitable for use in treating disorders of the cardiovascular system, including but not limited to elevated blood pressure.

In another preferred embodiment, the medicament may be used to treat or prevent vascular damage and/or neuropathy in diabetic subjects.

In yet another preferred embodiment, the condition to be treated is characterized by having an inflammatory component. In one particularly preferred embodiment, the medicament is used to treat and/or prevent vascular inflammation.

In a still further preferred embodiment, the medicament of the present invention may be used to prevent the onset of neoplastic conditions.

In some embodiments, said composition further comprises one or more of the additional active components mentioned hereinabove, such as additional carotenoids, polyphenols (such as camosic acid), vitamin E, phytosterols and soluble tomato solids (e.g. in the form of a clear tomato concentrate (CTC) prepared by concentrating the serum obtained from tomatoes following pulp-serum separation).

In a further aspect, the present invention is directed to the use of the above-defined reduced-lycopene tomato-derived composition as a topical medicament. In one preferred embodiment of this aspect, the topical medicament is a cosmetic or cosmeceutical preparation, and may be prepared in the form of a cream, lotion, ointment, gel and so on.

In one preferred embodiment, said cosmetic or cosmeceutical agent is used as sunscreen, wherein the cosmeceutical composition may also additionally comprise conventional sunscreen agents, including but not limited to, PABA, zinc oxide, titanium oxide, cinoxate, Padimate O and Phenylbenzimidazole sulfonic acid.

In another preferred embodiment, the tomato-derived composition of the present invention is used as an anti-aging agent. In some embodiments of the invention, the above-defined tomato composition is used as the sole active agent, in other cases, the cosmetic or cosmeceutical agent comprises an additional active component.

In another preferred embodiment, the present invention is directed to the use of the above-defined reduced-lycopene tomato-derived composition as a systemically-administered medicament, including, but not limited to, medicaments for oral delivery and medicaments suitable for parenteral delivery. In addition, the composition of the present invention may be used as a medicament suitable for topical administration.

### Brief description of the drawings

**Fig. 1** schematically illustrates a process for preparing a low-lycopene content tomato composition.
**Fig. 2** graphically illustrates the increase in NO levels in vascular endothelial cells, following treatment with the low-lycopene composition of the present invention.
**Fig. 3** graphically illustrates the increase in peNOS induction in vascular endothelial cells, following treatment with the low-lycopene composition of the present invention.
**Fig. 4** graphically depicts the increase in ARE activity in LNCaP prostate cancer cells caused by the low-lycopene composition of the present invention.
**Fig. 5** graphically depicts the increase in ARE activity in T47D mammary cancer cells caused by the low-lycopene composition of the present invention.
**Fig. 6** graphically depicts the inhibition of the NFkB transcription system in T47D mammary cancer cells that is caused by the low-lycopene composition of the present invention.

### Detailed description of preferred embodiments

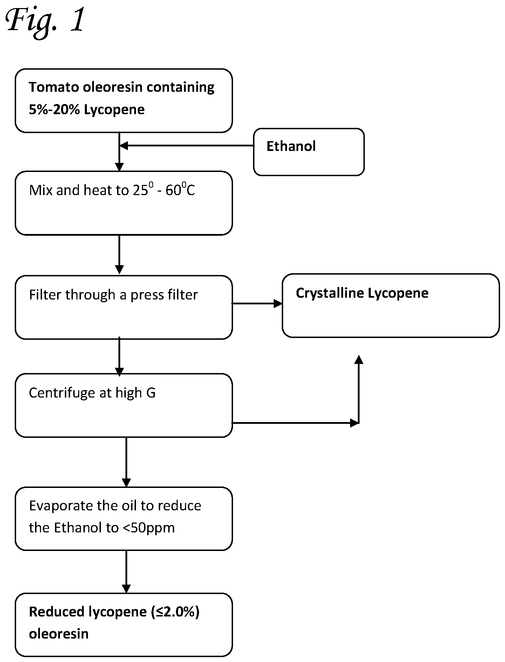

As disclosed hereinabove, the present invention provides composition comprising a reduced lycopene tomato product, wherein a general scheme of the process for manufacturing said composition is summarized in Fig. 1 and comprises the steps of:
*Step 1*: Providing a tomato product comprising 5% - 20% Lycopene (for example, an oleoresin, as described in US 5,837,311, or tomato peels).
*Step 2:* Adding a solvent or solvent mixture which dissolve the lipids and not the lycopene such as ethanol or isopropanol or acetone at a ratio higher than 1:1 (solvent: tomato product), preferably at a ratio of 3:1 or 4:1.
*Step 3:* Heating the solvent/tomato extract mixture at a temperature of about 25° - 60°C.
*Step 4:* Separating crystalline material from the solvent by means of filtering or centrifugal separation.
*Step 5:* Optionally incorporating the crystals, which contain approximately 70% lycopene, into colorant formulations.
*Step 6:* Partially evaporating the solvent (preferably leaving a liquor having less than 50 ppm solvent), which may then be recycled for use in step 2.
*Step* 7: Standardizing the resultant liquor to the required concentration, by adding the feed composition.

### Pharmaceutical Compositions

Although the active agents, lycopene and phytoene/phytofluene, optionally in combinations with other carotenoids, and/or additional agents, can be administered alone, it is contemplated that these compounds will be administered in a pharmaceutical composition containing the active ingredients together with a pharmaceutically acceptable carrier or excipient.

Pharmaceutical compositions for use in accordance with the present invention can be formulated in conventional manner using one or more physiologically acceptable carriers comprising excipients and auxiliaries, which facilitate processing of the active compounds into preparations which can be used pharmaceutically. The active agents are formulated as pharmaceutical compositions and administered to a mammalian subject, such as a human patient in a variety of forms such as liquid, solid, and semisolid. The pharmaceutical compositions can be administered to a subject by any method known to a person skilled in the art, such as orally, topically, parenterally, transmucosally, transdermal, intramuscularly, intravenously, intradermally, subcutaneously, intraperitonealy, intraventricularly, intracranially or intratumorally. For oral administration, the compounds can be formulated by combining the active compounds with pharmaceutically acceptable carriers known in the art. The compositions can be formulated in any solid or liquid dosage form known in the art, including but not limited to, tablet, caplet , capsule, microcapsule, pellet, pill, powder, syrup, gel, slurry, granule, suspension, dispersion, emulsion, liquid, solution, dragee, bead and beadlet. The oral compositions can be formulated as immediate release formulations, or as controlled or sustained release formulations allowing for extended release of the active ingredient(s) over a predetermined time period.

Suitable excipients for solid formulations include but are not limited to fillers such as sugars, including lactose, sucrose, mannitol, or sorbitol; starch based excipients such as maize starch, wheat starch, rice starch, potato starch and the like, gelatin, gum tragacanth, cellulose based excipients as microcrystalline cellulose, carboxymethylcellulose, hydroxymethylcellulose, hydro xyethylcellulose, methylhydroxypropylcellulose, hydroxypropylcellulose and the like. Polymers such as polyvinylpyrrolidone (PVP) and cross-lined PVP can also be used. In addition, the compositions may further comprise binders (e.g. acacia, cornstarch, gelatin, carbomer, ethyl cellulose, guar gum, hydroxypropyl cellulose, hydroxypropyl methyl cellulose, povidone), disintegrating agents (e.g. cornstarch, potato starch, alginic acid, silicon dioxide, croscarmelose sodium, crospovidone, guar gum, sodium starch glycolate), surfactants (e.g. sodium lauryl sulfate), and lubricants (e.g. stearic acid, magnesium stearate, polyethylene glycol, sodium lauryl sulfate).

For liquid formulations, pharmaceutically acceptable carriers may be aqueous or non-aqueous solutions, suspensions, emulsions or oils. Examples of non-aqueous solvents are propylene glycol, polyethylene glycol, and injectable organic esters. Aqueous carriers include water, alcoholic/aqueous solutions, emulsions or suspensions, including saline and buffered media. Examples of oils include but are not limited to petroleum, animal, vegetable, or synthetic origin, for example, peanut oil, soybean oil, mineral oil, olive oil, sunflower oil, and fish-liver oil.

Preferred oral pharmaceutical compositions include capsules made of gelatin as well as soft, sealed capsules made of gelatin and a plasticizer, such as glycerol or sorbitol. In soft capsules, the active compounds may be dissolved or suspended in suitable liquids, such as fatty oils, liquid paraffin, or liquid polyethylene glycols. In addition, stabilizers may be added. In certain preferred embodiments the capsules exclude components of animal origin and are acceptable for vegetarians and vegans.

Soft gelatin capsules and methods of preparing them are known in the art. Non- limiting examples can be found in US Patent Nos. 6,217,902; 6,258,380; 5,916,591, and 4,891, 229. Other acceptable excipients and additives known to the person with skill in the art may be included in the compositions of the present invention, for example stabilizers, solubilizers, tonicity enhancing agents, buffer substances, preservatives, thickeners, complexing agents and other excipients, as well as additional therapeutic agents.

A solubilizer can be for example, tyloxapol, fatty acid glycerol polyethylene glycol esters, fatty acid polyethylene glycol esters, polyethylene glycols, glycerol ethers or mixtures of those compounds. A specific example of a solubilizer is a polyoxyethylated castor oil for example, the commercial products Cremophor ® or Cremophor ® RH40. Another example of a solubilizer is tyloxapol. The concentration used depends especially on the concentration of the active ingredient. The amount added is typically sufficient to solubilize the active ingredient. For example, the concentration of the solubilizer is from 0.1 to 5000 times the concentration of the active ingredient

Examples of buffer substances are acetate, ascorbate, borate, hydrogen carbonate/carbonate, citrate, gluconate, lactate, phosphate, propionate and TRIS (tromethamine) buffers. The amount of buffer substance added is, for example, that necessary to ensure and maintain a physiologically tolerable pH range. The pH range is typically in the range of from 5 to 9, preferably from 5.2 to 8.5.

The compositions of the present invention may comprise further non-toxic excipients, such as, for example, emulsifiers, wetting agents or fillers, such as, for example, the polyethylene glycols (PEG200, 300, 400 and 600) or Carbowax ® (CarbowaxlOOO, 1500, 4000, 6000 and 10000). Other excipients that may be used if desired are listed below but they are not intended to limit in any way the scope of the possible excipients. They can be complexing agents, such as disodium-EDTA or EDTA 5 antioxidants, such as ascorbic acid, acetylcysteine, cysteine, sodium hydrogen sulfite, butyl-hydroxyanisole, butyl- hydroxytoluene; stabilizers, such thiourea, thiosorbitol, sodium dioctyl sulfosuccinate or monothioglycerol; or other excipients, such as, for example, lauric acid sorbitol ester, Methanol amine oleate or palmitic acid ester.

The amount of a composition to be administered will, of course, depend on many factors including the subject being treated, the severity of the affliction, the manner of administration, and the judgment of the prescribing physician. However, the dose employed will generally depend on a number of factors, including the age and sex of the patient, and the severity of the disease being treated.

Preferably, the preparations are in unit dosage form, intended for oral administration. In such form, the preparation is subdivided into unit doses containing appropriate quantities of the active components. The unit dosage form can be a packaged preparation, the package containing discrete quantities of preparation, for example, tablets, capsules, and powders in vials or ampoules. The unit dosage form can also be a capsule, cachet, or tablet itself or it can be the appropriate number of any of these in packaged form.

The dosing schedule of the compositions of the present invention can vary according to the particular application and the potency of the active ingredients. Determination of the proper dosage is within the skill of the art. For convenience, a single daily dose is preferred. Alternatively, the total daily dosage may be divided and administered in portions during the day such as twice daily, thrice daily and the like. Biweekly, weekly, bimonthly and monthly administration are also contemplated

The present invention will be further described in the following Examples, which are brought for illustrative purposes only, and do not limit the scope of the invention in any way.

### Preparative Example 1

### Process for producing the low-lycopene concentration tomato-derived composition of the present invention

The low-lycopene concentration composition of the present invention was prepared in accordance with the following process steps:
1. A tomato oleoresin having the following composition was prepared according to the process disclosed in US 5,837,311:
   10.1% lycopene
   1.7% phytoene and phytofluene
   2.5% phytosterols
   2.5% vitamin E
   82.7% free fatty acids
   0.5% water
   (It is to be noted that this preparation may be purchased under the trade name of Lyc-O-Mato®, manufactured by LycoRed Ltd. of Be'er Sheva, Israel.)
2. 100 ml of the tomato oleoresin prepared according to step 1 was added to a mixing vessel containing 300 ml of 98% ethanol, and mixed at 40°C for 0.5 hour.
3. A press filter was used in order to separate the crystalline material from the ethanol solvent. This separation yielded 10.94g of crystalline material comprising 85% lycopene and 15% fatty acids.
4. The filtrate from step 3 was then subjected to evaporation, in order to remove most of the ethanol, leaving 89g of a liquor (which is the low-lycopene product of the present invention) having the following composition:
   2.0% lycopene
   2.2% phytoene and phytofluene
   2.8% phytosterols
   2.8% vitamin E

### Experimental Examples

### General Methods

### Cell culture and treatments

Primary human umbilical vein endothelial cells (HUVECs) were isolated from umbilical cords. The cells were harvested by 0.1% collagenase treatment (Worthington, Lakewood, NJ, USA). HUVECs were grown on 0.2% gelatin pre-coated tissue-culture flasks (Corning, Cole-Parmer, Vernon Hills, IL, USA) in M-199 medium with 20% BCS and other supplements as described for EA.hy926. EA.hy926 cells were used until passage 37, HUVECs at passage 3-8. U937 monocytes were cultured in RPMI 1640 medium with 10% BCS, L-glutamine and antibiotics as specified earlier. For most experiments the endothelial cells at 70-90% confluence were starved in 5% BCS DMEM/ M199 medium for 24 hours and pre-incubated with vehicle or carotenoids solution in 5% BCS DMEM/ M199 for 18 to 24 hours, and activated with 10 ng/mL TNF-α (Peprotech, Rocky Hill, NJ, USA) for specified times.

### Carotenoid solutions

Stock solutions (400uM) of oleoresin (Lycored Natural Products Ltd., Beer-Sheva, Israel), containing either 6% or 7% lycopene, 0.1% β-carotene, 1% vitamin E and polyphenols, and the low-lycopene composition of the present invention (as obtained according to Preparative Example 1, above) were prepared in fresh tetrahydrofuran (THF) containing 0.025% butylated hydrohytoluene (BHT) (both from Sigma, St. Louis, MO, USA) as an antioxidant, and were added to the culture medium under N₂ stream and reduced lighting.

### Experimental Example 1

### The effect of the the low-lycopene concentration tomato-derived composition of the present invention on the expression of nitric oxide (NO)

NO is a key signaling molecule in many biological processes and tissues. In the case of vascular tissues, the endothelium uses nitric oxide to signal the surrounding smooth muscle to relax, thus resulting in vasodilation and increased blood flow. NO further plays a role in vascular function by inhibiting vascular smooth muscle growth, platelet aggregation, and leukocyte adhesion to the endothelium. In many cases of human disease that are characterized by the presence of cardiovascular lesions (such as atherosclerosis, diabetes, or hypertension), impaired NO pathway function may often be found.

In the present study, a cultured human coronary endothelial cell model was used to assess the effect of the low-lycopene (2%) concentration composition of the present invention on the induction of NO. This was achieved both by the direct measurement of NO production by the cultured cells, and by assay of the activity of the endothelial cell NO synthase enzyme, peNOS. The results achieved with the composition of the present invention were compared with results obtained with the use of a high-lycopene tomato-derived composition, 7% Lyc-O-Mato (manufactured and supplied by LycoRed Ltd., Be'er Sheva, Israel).

### Methods and materials:

The low-lycopene (2%) composition of the present invention (prepared as described hereinabove and the various control solutions (medium, THF and 7% Lyc-O-Mato) were added to confluent cultured human coronary endothelial cells (plated in 2 ml endothelial cell basal medium *per* 60 mm dish) for 24 hours, following which the supernatant culture fluid was collected.

### Nitrate and Nitrite Analysis

Nitrite and nitrate (NOx), stable oxidized products of nitric oxide, were measured in the culture medium collected 24 h following the exposure to carotenoids using Greiss reagent, using the method described in Miranda KM, Espey MG and Wink DA, 2001 (Nitric Oxide 5: 62-71; "A rapid, simple spectrophotometric method for simultaneous detection of nitrate and nitrite").

Experiments were performed at room temperature or at 37°C in a warm room, as noted.

### peNOS analysis

Expression of the endothelial NO synthase enzyme, peNOS was assessed using Western blot analysis, as well known in the art. In brief, the protein content of an endothelial cell lysate was quantified using a BCA protein assay kit (Pierce, Rockford, IL, USA. Equal quantities of cell proteins were separated by 7.5% SDS-PAGE and blotted to nitrocellulose membrane. After blocking and incubation with the primary peNOS antibody, the relative changes in the proteins content were quantified using densitometry in a reflectance mode.

### RESULTS

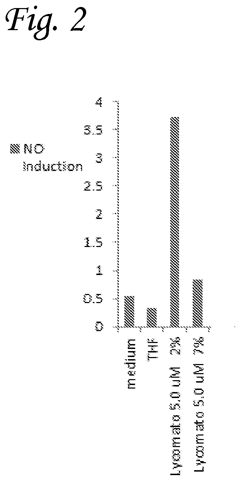

As shown in Fig. 2, when used at a concentration of 5.0 µM, the low-lycopene (2%) composition of the present invention caused a significant increase in the level of NO induction, when compared with both the medium and THF controls. Unexpectedly, 7% Lyc-O-Mato, while also causing a measurable increase in NO expression, was far less active in this regard (by a factor of approximately four) than the composition of the present invention.

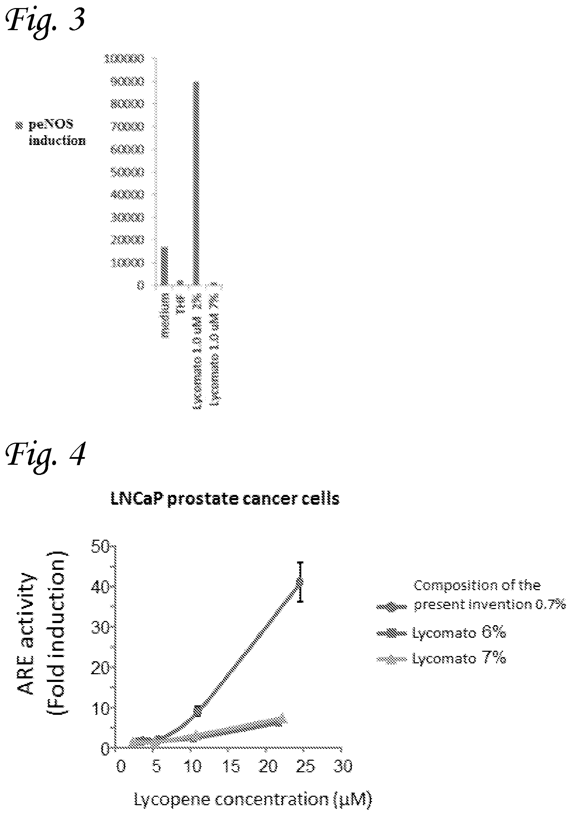

Fig. 3 presents the results obtained from the Western blot analysis of peNOS levels in the cultured endothelial cells. Thus, it may be seen from this figure that the low lycopene (2%) composition of the present invention (used at 1.0 µM) caused highly significant induction of peNOS, as compared to both the medium and THF controls. This result differs markedly from that obtained using 7% Lyc-O-Mato (1.0 µM) which did not cause any increase in peNOS expression in comparison with untreated cells.

It may be concluded from these results that the low-lycopene concentration composition of the present invention is capable of significantly increasing the induction of NO in vascular endothelial cells, and that this effect is at least in part caused by increased expression of NO synthase enzyme in said cells. In view of these results, it may be concluded that the composition of the present system has activity of relevance that may be utilized in the treatment and prevention of various types of cardiovascular disease.

### Experimental Example 2

### Stimulation of the ARE transcription system by the low-lycopene concentration tomato-derived composition of the present invention

Antioxidant responsive elements (AREs) are identified in gene promoters and mediate a transcriptional induction of a battery of genes which comprise a chemoprotective response system. Said system is essential for resistance against a broad set of carcinogens. In addition, the activation of cytoprotective ARE-regulated genes can suppress inflammatory responses, whereas decreased expression of these genes results in autoimmune disease and enhanced inflammatory responses to oxidant insults. Therefore this ARE model is used as well in order to determine the anti-inflammatory effects of the tested compositions.

The aim of the present study was to assess the activity of a reduced-lycopene (0.7% w/w) modified oleoresin composition of the present invention, in the ARE system. The results obtained using this composition were compared with those obtained using LycoMato (containing 6% or 7% lycopene), present in equal molar concentrations of lycopene

### Methods and materials:

A cell culture model, using both mammary (T47D) and prostate (LnCAP) cancer cells, was used to assess the activity of a low lycopene composition of the present invention, prepared as disclosed hereinabove, on the ARE system. Said low lycopene composition comprised 0.7% lycopene, 1.7% phytoene/phytofluene, 2.4% phytosterols and 2.5% vitamin E. Two commercially-available high-lycopene compositions (LycoMato 6% and LycoMato 7%; manufactured by LycoRed Ltd., Be'er Sheva, Israel) were also tested, for the purpose of comparison.

### Cell culture

LNCaP, human prostate cancer cells were purchased from American Type Culture Collection (Manassas, VA, USA) and grown in RPMI 1640 medium containing sodium pyruvate (0.11 mg/ml) and DHT (10-9M). To each medium, penicillin (100 units/ml), streptomycin (0.1 mg/ml), nystatin (12.5 µg/ml), Hepes (10 mM), and 10% FCS were added.

T47D, a human mammary cancer cell line, was kindly provided by Dr. Iafa Keydar (Tel Aviv University, Israel). T47D cells were grown in DMEM containing insulin(0.6 µg/ml or 6 µg/ml).

### Transient transfection and ARE reporter gene assay

T47D cells were transfected using jet PEI reagent (Polyplus Transfection, Illkrich, France) in 24-well plates (100,000 cells per well). Cells were rinsed once with the appropriate culture medium without serum, followed by the addition of 0.45 ml of medium containing 3% DCC-FCS and 50 µl of a mixture containing DNA and jetPEI reagent at a charge ratio of 1:5. The total amount of DNA was 0.25 µg containing 0.2 µg reporter and 0.05 µg Renilla luciferase. The cells were then incubated for 4-6 h at 37 °C in 95% air/5% CO2. Medium was replaced with one supplemented with 3% DCC-FCS plus the test compounds, and cells were incubated for another 16 h.

LNCaP cells were transfected using the jetPEI reagent. Cells (70,000) were seeded in 1 ml medium without phenol red containing 3% DCCFCS. On the next day, 500 µl of medium was removed and 50 µl of a mixture containing DNA and jetPEI reagent at a charge ratio of 1:10 was added. The total amount of DNA was 0.2 µg containing 0.16 µg reporter and 0.04 µg Renilla luciferase vectors. Cells were incubated for 4-6 h followed by addition of the test compounds for 24 h.

### Luciferase reporter assay for ARE activity evaluation

Cell extracts were prepared for luciferase reporter assay (Dual Luciferase Reporter Assay (DLR™) System, Promega) according to the manufacturer's instructions. The Dual-Luciferase Reporter Assay System provides an efficient means of performing dual-reporter assays. In the DLR™ Assay, the activities of firefly (*Photinus pyralis*) and *Renilla* (*Renilla reniformis,* also known as sea pansy) luciferases are measured sequentially from a single sample. The firefly luciferase reporter is measured first by adding Luciferase Assay Reagent II (LAR II) to generate a stabilized luminescent signal. After quantifying the firefly luminescence, this reaction is quenched, and the *Renilla* luciferase reaction is simultaneously initiated by adding Stop & Glo® Reagent to the same tube. The Stop & Glo® Reagent also produces a stabilized signal from the *Renilla* luciferase, which decays slowly over the course of the measurement.

### RESULTS

As demonstrated in Fig. 4, the low-lycopene composition of the present invention induces significantly greater ARE activity than Lyc-O-Mato in LNCaP prostate cancer cells, when compared at equal molar concentrations of lycopene. The observed effect is dose-dependent. Above the inflection point of the curve (around 10 µM) the differences between the presently-claimed composition and Lyc-O-Mato, with regard to ARE induction are more significant.

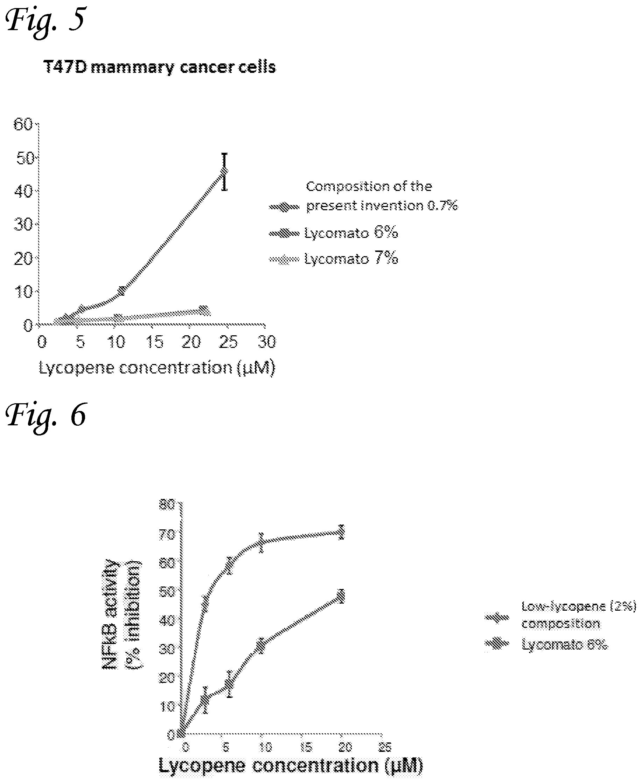

As shown in Fig. 5, similar results are also obtained when the composition of the present invention is added to T47D mammary cancer cells. Thus, as in the case of the results obtained with the prostate cancer cells, the presently-claimed composition caused significantly greater activity in the ARE system than was seen with the Lyc-O-Mato controls.

It may be concluded from these results that the low-lycopene concentration composition of the present invention is a highly active inducer of ARE activity. Said composition therefore has the potential for influencing important cellular machinery that are of great importance in both the prevention of cancer and the reduction of inflammation.

### Experimental Example 3

### Inhibition of the NFkB transcription system by the low-lycopene concentration tomato-derived composition of the present invention

Expression of inflammatory cytokines as well enzyme protein expression can be regulated by the activation of the transcription factor nuclear factor-kappa B (NFκB), which is critically involved in several aspects of the pathogenesis chronic inflammatory diseases. NFκB is activated as a consequence of phosphorylation, ubiquitination, and subsequent proteolytic degradation of the IκB protein through activation of IκB kinase (IKK). The liberated NFκB translocates into nuclei and binds to motifs in the promoters of pro-inflammatory genes such as inducible nitric oxide synthase (iNOS) and of cyclooxygenase 2 (COX2) TNF-α, and IL-1β, leading to the induction of their mRNA expression. Many of the anti-inflammatory drugs previously developed have been shown to suppress the expression of these genes by inhibiting the NFκB activation pathway. Thus, an NFκB inhibitor may be useful as a potential therapeutic drug in clinical applications for regulating the inflammation associated human diseases.

The aim of this study was to investigate whether the low-lycopene composition of the present invention can inhibit the NFkB transcription system in T47D mammary cancer cells.

The results of this study are shown in Fig. 6. It may be seen from this figure that the low-lycopene composition of the present invention causes significantly greater inhibition of the NFkB transcription system in T47D mammary cancer cells than the 6% Lyc-O-Mato control. These results provide further evidence for the high-level anti-inflammatory activity of the low-lycopene composition for the present invention.

## Claims

1. A composition comprising lycopene, one or both of phytoene and phytofluene, and phytosterols, wherein the concentration of lycopene is in the range of 0.3% - 2% (w/w), wherein the weight ratio of said lycopene to one or both of phytoene and phytofluene is in the range of 1:1 to 1:2.5 and wherein the concentration of phytosterols is at least 2% (w/w).

2. The composition according to claim 1, further comprising vitamin E at a concentration of at least 2% (w/w).

3. The composition according to claim 1, further comprising one or more additional components selected from the group consisting of additional carotenoids, vitamin E, polyphenols and soluble tomato solids.

4. The composition according to claim 1, further comprising beta-carotene.

5. The composition according to claim 1, further comprising carnosic acid.

6. The composition according to claim 1, further comprising soluble tomato solids.

7. The composition according to claim 1, wherein said composition is prepared from tomatoes.

8. A process for preparing a composition comprising lycopene, one or both of phytoene and phytofluene, and phytosterols as defined in claim 1, said process comprising the steps of:
a) Providing a tomato product comprising 5-20% (w/w) lycopene;
b) Extracting carotenoid-rich material from said tomato product using ethanol, isopropanol, ethyl acetate or acetone at a ratio higher than 1:1 (solvent: tomato extract) as the extraction solvent;
c) Heating the solvent/tomato extract mixture at a temperature of about 25° - 60°C;
d) Separating crystalline material from the solvent by means of filtering or centrifugal separation; and
e) Evaporating the supernatant obtained in step (d), thereby obtaining said tomato composition

9. The process according to claim 8, wherein the solvent is ethanol.

10. The process according to claim 8, wherein the tomato product is an oleoresin.

11. The process according to claim 8, wherein the tomato product comprises tomato peels.

12. The composition according to any one of claims 1 to 7 for use as a medicament.

13. The composition according to any one of claims 1 to 7 for use as a cosmetic or cosmeceutical agent.

## Patentansprüche

1. Zusammensetzung, umfassend Lycopin, Phytoen oder Phytofluen oder beides, und Phytosterine, wobei die Konzentration von Lycopin sich im Bereich von 0,3 % bis 2 Gew.-% bewegt, wobei das Gewichtsverhältnis des besagten Lycopin zu Phytoen oder Phytofluen oder beides sich im Bereich von 1:1 bis 1:2,5 bewegt und wobei die Konzentration von Phytosterinen zumindest 2 Gew.-% beträgt.

2. Zusammensetzung nach Anspruch 1, ferner umfassend Vitamin E mit einer Konzentration von zumindest 2 Gew.-%.

3. Zusammensetzung nach Anspruch 1, ferner umfassend eine oder mehrere zusätzliche Komponenten, ausgewählt aus der Gruppe, bestehend aus zusätzlichen Carotinoiden, Vitamin E, Polyphenolen und löslichen Tomatenfeststoffen.

4. Zusammensetzung nach Anspruch 1, ferner umfassend Beta-Carotin.

5. Zusammensetzung nach Anspruch 1, ferner umfassend Carnosolsäure.

6. Zusammensetzung nach Anspruch 1, ferner umfassend lösliche Tomatenfeststoffe.

7. Zusammensetzung nach Anspruch 1, wobei besagte Zusammensetzung aus Tomaten hergestellt wird.

8. Verfahren zum Herstellen einer Zusammensetzung, umfassend Lycopin, Phytoen oder Phytofluen oder beides, und Phytosterole wie definiert in Anspruch 1, besagtes Verfahren umfassend die folgenden Schritte:
a) Bereitstellen eines Tomatenprodukts, umfassend 5 bis 20 Gew.-% Lycopin;
b) Extrahieren carotinoidreichen Materials aus besagtem Tomatenprodukt unter Nutzung von Ethanol, Isopropanol, Ethylacetat oder Aceton mit einem Verhältnis höher als 1:1 (Lösungsmittel: Tomatenextrakt) als das Extraktionslösungsmittel;
c) Erhitzen des Lösungsmittels / der Tomatenextraktmischung bei einer Temperatur von etwa 25 ° bis 60 °C;
d) Trennen kristallinen Materials vom Lösungsmittel im Wege der Filterung oder Zentrifugalabscheidung; und
e) Verdampfen des in Schritt (d) erlangten Überstands, wodurch besagte Tomatenzusammensetzung erlangt wird.

9. Verfahren nach Anspruch 8, wobei das Lösungsmittel Ethanol ist.

10. Verfahren nach Anspruch 8, wobei das Tomatenprodukt ein Fettharz ist.

11. Verfahren nach Anspruch 8, wobei das Tomatenprodukt Tomatenschalen umfasst.

12. Zusammensetzung nach einem der Ansprüche 1 bis 7 zur Nutzung als ein Medikament.

13. Zusammensetzung nach einem der Ansprüche 1 bis 7 zur Nutzung als ein Kosmetikum oder kosmezeutischer Wirkstoff.

## Revendications

1. Composition comprenant du lycopène, un ou les deux phytoène et phytofluène, et phytostérols, dans laquelle la concentration de lycopène est comprise entre 0,3 % - 2 % (p/p), dans laquelle le rapport pondéral dudit lycopène à un ou aux deux phytoène et phytofluène est compris entre 1:1 à 1:2,5 et dans laquelle la concentration des phytostérols est d'au moins 2 % (p/p).

2. Composition selon la revendication 1, comprenant en outre de la vitamine E à une concentration d'au moins 2 % (p/p).

3. Composition selon la revendication 1, comprenant en outre un ou plusieurs composants supplémentaires sélectionnés parmi le groupe constitué de caroténoïdes supplémentaires, de vitamine E, de polyphénols et de solides de tomates solubles.

4. Composition selon la revendication 1, comprenant en outre de la bêta-carotène.

5. Composition selon la revendication 1, comprenant en outre de l'acide carnosic.

6. Composition selon la revendication 1, comprenant en outre des solides de tomates solubles.

7. Composition selon la revendication 1, dans laquelle ladite composition est préparée à partir de tomates.

8. Procédé de préparation d'une composition comprenant du lycopène, un ou les deux phytoène et phytofluène, et les phytostérols tels que définis dans la revendication 1, ledit procédé comprenant les étapes consistant à :
a) Fournir un produit à base de tomates comprenant 5-20 % (p/p) de lycopène ;
b) Extraire un matériau riche en caroténoïdes provenant dudit produit à base de tomates à l'aide d'éthanol, d'isopropanol, d'acétate d'éthyle ou d'acétone à un rapport supérieur à 1 :1 (solvant : extrait de tomate) comme solvant d'extraction ;
c) Chauffer le mélange solvant/tomate à une température d'environ 25°-60°C ;
d) Séparer le matériau cristallin du solvant par le filtrage ou la séparation centrifuge ; et
e) Évaporer le surnageant obtenu à l'étape (d), ce qui permet d'obtenir ladite composition de tomates

9. Procédé selon la revendication 8, dans lequel le solvant est l'éthanol.

10. Procédé selon la revendication 8, dans lequel le produit de tomates est une oléorésine.

11. Procédé selon la revendication 8, dans lequel le produit de tomates comprend des pelures de tomates.

12. Composition selon l'une quelconque des revendications 1 à 7 à utiliser comme médicament.

13. Composition selon l'une quelconque des revendications 1 à 7 à utiliser comme agent cosmétique ou cosméceutique.
